# EUROPÄISCHE PATENTSCHRIFT

(11) **EP 1 095 656 B1**
(45) Veröffentlichungstag und Bekanntmachung des Hinweises auf die Patenterteilung: **20.08.2003**
(21) Anmeldenummer: 01100944.6
(22) Anmeldetag: 14.03.1996
(51) Int. Cl.: A61K 31/55, A61P 25/28, A61P 25/30

(54) **Pharmazeutische Zubereitung mit dem Cholinesterase-Hemmstoff Galanthamin**
Pharmaceutical preparation comprising Galanthamine as cholinesterase inhibitor
Préparation pharmaceutique comprenant de galanthamine comme inhibiteur de la cholinesterase

(30) Priorität: 17.03.1995 DE 19509663
(43) Veröffentlichungstag der Anmeldung: 02.05.2001
(62) Teilanmeldung aus: 96907470.7
(73) Patentinhaber: LTS LOHMANN Therapie-Systeme AG, 56626 Andernach (DE)
(72) Erfinder: Hille, Thomas, 56567 Neuwied (DE); Hoffmann, Hans-Rainer, 56564 Neuwied (DE); Kreh, Mirko, 64850 Schaafheim (DE); Matusch, Rudolf, 35041 Marburg (DE)
(74) Vertreter: Flaccus, Rolf-Dieter, Dr.

(56) Entgegenhaltungen:
- EP-A- 0 236 684
- EP-A- 0 515 301
- DE-A- 4 010 079
- DE-B- 1 193 061
- DE-C- 4 301 782
- DE-C- 4 301 783
- COZANITIS D A ET AL: "STUDY OF THE ANALGESIC EFFECTS OF GALANTHAMINE A CHOLINESTERASE INHIBITOR" ARCHIVES INTERNATIONALES PHARMACODYNAMIE ET DE THERAPIE,US,NEW YORK, NY, Bd. 266, Nr. 1, November 1983 (1983-11), Seiten 229-238, XP000892208 ISSN: 0003-9780
- J. BASTIDA ET AL: "Alkaloids from Narcissus confusus" PHYTOCHEMISTRY, Bd. 26, Nr. 5, 1987, Seiten 1519-1524, XP000571959

## Beschreibung

Die vorliegende Erfindung betrifft pharmazeutische Zubereitungen, die den reversibel wirkenden Cholinesterasehemmstoff Galanthamin enthalten. Die Erfindung betrifft ferner. die Verwendung von Galanthamin zur Herstellung von Medikamenten zur Behandlung von Krankheitszuständen, die durch die Verabreichung eines reversibel wirkenden Cholinesterase-Hemmstoffs therapierbar sind.

Galanthamin (4a,5,9,10,11,12-Hexahydro-3-methoxy-11-methyl-6-H-benzofuro-(3a,3,2-ef)-(2)-benzazepin-6-ol) ist ein tetracyclisches Alkaloid, das aufgrund seiner pharmakologischen Eigenschaften zur Gruppe der reversibel wirkenden Cholinesterasehemmstoffen gehört und in seinen Wirkungen dem Physostigmin und dem Neostigmin nahesteht. Es besitzt jedoch auch eigene spezifische Eigenschaften, wie beispielsweise mit Morphin vergleichbare stark analgetische Wirkungen. Als Cholinesterasehemmer besitzt Galanthamin aufgrund seiner im Vergleich zu Physostigmin und Neostigmin geringeren Toxizität eine drei- bis sechsmal größere therapeutische Breite. Dieser Vorteil wiegt seine dosisbezogene etwas geringere Cholinesterasehemmwirkung auf. Galanthamin wird bei Poliomyelitis und verschiedenen Erkrankungen des Nervensystems eingesetzt, hauptsächlich jedoch bei der Behandlung des Engwinkelglaukoms und als Antidot nach Curare Applikationen. Versuchsweise wird Galanthamin bei der Alzheimerschen Krankheit eingesetzt. In jüngster Zeit wurde auch die Behandlung der Alkohol- und Nikotinabhängigkeit beschrieben (DE-OS 40 10 079, DE-OS 43 01 782).

Sowohl die Therapie der Alzheimerschen Krankheit, der Alkohol- und Nikotinabhängigkeit als auch des Engwinkelglaukoms erfordern langwirksame, den besonderen Umständen angepasste Arzneiformen. Als solche bietet sich bei der Behandlung des Engwinkelglaukoms eine Augensalbe an. Schwierige Therapieschemata und Dauerinfusionen kommen aus naheliegenden Gründen zur Behandlung der Alzheimerschen Krankheit, der Alkohol- oder Nikotinabhängigkeit nicht in Frage. Bei diesen Erkrankungen bietet sich vielmehr ein transdermales therapeutisches System (TTS) an, wie es beispielsweise in der DE-OS 43 01 783 beschrieben wird. Weder die intakte Haut noch die Cornea des Auges gestatten eine Resorption von Wirkstoffsalzen. Galanthaminhydrochlorid oder Galanthaminhydrobromid können daher bei der Therapie des Engwinkelglaukoms, der Alzheimerschen Krankheit oder der Alkohol- oder Nikotinabhängigkeit mit Salben oder dem TTS nicht verwendet werden. Aus diesem Grund muß also die reine Galanthaminbase eingesetzt werden.

Aufgrund seiner komplizierten tetracyclischen Struktur mit drei optisch aktiven Kohlenstoffatomen ist eine wirtschaftliche Synthese der Galanthaminbase nicht möglich. Galanthamin wird daher üblicherweise aus Pflanzen der Amaryllidaceen, beispielsweise aus Galanthusarten, wie dem Schneeglöckchen oder Leucojum aestivum, isoliert. Diese Pflanzen haben zwar den Vorteil, Galanthamin in Konzentrationen von bis zu 0,3 %, bei gleichzeitig geringem Anteil an Nebenalkaloiden zu enthalten, so daß das in der DE-PS 11 93 061 beschriebene Extraktionsverfahren Anwendung finden kann. Sowohl die Galanthusarten als auch Leucojum aestivum stehen jedoch unter Naturschutz. Zum anderen verwendet das in der DE-PS 11 93 061 beschriebene Extraktionsverfahren vorzugsweise chlorierte Kohlenwasserstoffe, die aus toxikologischen Gründen in Verruf geraten sind. Die Arzneibücher der westlichen Welt fordern daher den Restgehalt an chlorierten Kohlenwasserstoffen auf < 10 ppm zu beschränken. Der Einsatz chlorierter Kohlenwasserstoffe sollte folglich bei der Bereitstellung von Arzneistoffen möglichst vermieden werden. Darüber hinaus muß bei dem bekannten Verfahren der Lösungsmittelextrakt an Aluminiumoxyd adsorbiert werden, um die Abtrennung der Harzstoffe und Nebenalkaloide zu gewährleisten. Aus der nach dem Abfiltieren des Aluminiumoxyds erhaltenen Lösung wird das Galanthamin dann über das Galanthaminhydrobromid gereinigt, mit der damit verbundenen Entsorgung von Halogensalzen. Für den Einsatz in Salben und dem TTS muß dann die Galanthaminbase auch noch aus diesem Galanthaminhydrobromid freigesetzt werden.

Aufgabe der vorliegenden Erfindung ist die Bereitstellung von pharmazeutischen Zubereitungen, welche einen reversibel wirkenden Cholinesterase-Hemmstoff enthalten, und die für die Behandlung von Krankheitszuständen verwendet werden können.
Diese Aufgabe wird erfindungsgemäß durch pharmazeutische Zubereitungen nach den Ansprüchen 1 bis 3 gelöst, sowie durch die Ansprüche 4 bis 8.

Gegenstand der Erfindung ist auch ein Verfahren zur Isolierung von Galanthamin aus biologischem Material, das aus landwirtschaftlich angebauten Amaryllidaceenarten oder aus solchen, die allgemein als "Unkraut" gelten und nicht unter Naturschutz stehen, vorzugsweise aus den Zwiebeln dieser Pflanzen, gewonnen wird. Zu diesen Amaryllidaceen zählen beispielsweise Narzissen bzw. Crinumarten. Besonders geeignet sind Narcissus pseudonarcissus "Carlton" oder die asiatische Kletterpflanze Crinum amabile. Obwohl diese Pflanzen nur etwa ein Zehntel der Galanthaminmenge der unter Naturschutz stehenden Pflanzen und darüber hinaus auch noch bis zu zwölf Nebenalkaloide enthalten-, gelingt es überraschenderweise mit dem erfindungsgemäßen Verfahren hieraus die Galanthaminbase in für Arzneimittel geeigneter Reinheit zu isolieren.

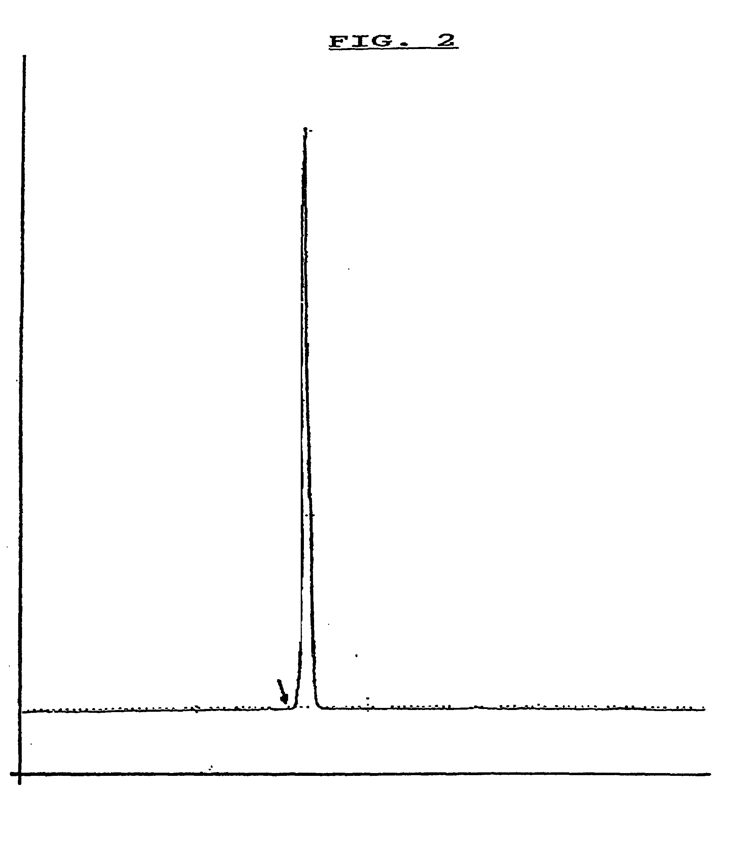

Erfindungsgemäß wird Galanthamin enthaltendes, biologisches Material, das vorzugsweise zerkleinert und mit Alkalipulver, vorzugsweise mit Natriumhydroxid-Plätzchen, Soda, Pottasche oder ähnlichen Salzen, die geeignet sind, Basen aus biologischem Material freizusetzen und zur Darstellung pharmokologischer Wirkstoffe geeignet sind, vermischt wird, mit toxikologisch unbedenklichen organischen Lösungsmitteln extrahiert und das Galanthamin aus diesem Extrakt durch Flüssig/Flüssig Extraktion gereinigt. Von besonderer Bedeutung für den Erfolg des erfindungsgemäßen Verfahrens ist die Einhaltung des pH-Wertes in der ersten Stufe der Flüssig/Flüssig-Extraktion. Die Flüssig/Flüssig Extraktion wird in einer ersten Stufe bei einem pH-Wert von etwa 4 und in einer zweiten Stufe bei einem pH-Wert von etwa 9 durchgeführt. Zur Einstellung des pH-Wertes kann nebem konzentriertem Ammoniak auch Soda-Lösung oder eine andere Base eingesetzt werden. Als toxikologisch unbedenkliches organisches Lösungsmittel wird Diethylether oder Spezialbenzin eingesetzt. Vorzugsweise wird Spezialbenzin verwendet, da sich damit bereits eine gewisse Vorreinigung erzielen läßt. Das Lösungsmittel wird entfernt, das Galanthamin aus einem geeigneten Lösungsmittel, vorzugsweise aus Isopropanol umkristallisiert. Man erhält die weiße Galanthaminbase mit einem Schmelzpunkt von 129 bis 130°C. Die Reinheit des so erhaltenen Galanthamins zeigt sich im HPLC-Chromatogramm (Fig.2).

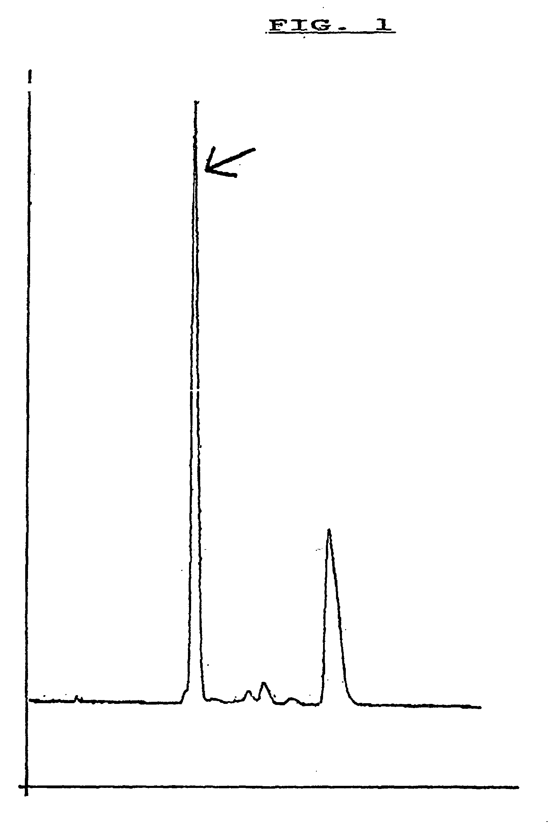

Dieses Ergebnis verblüfft umso mehr, als sich das bisher verwendete Verfahren aus der DE-PS 11 93 061 zur Isolierung des Galanthamins aus biologischem Material, das wenig Galanthamin und bis zu 12 Nebenalkaloide enthält, als ungeeignet herausstellte. Mit dem in der DE-PS 11 93 061 beschriebenen Verfahren entsteht eine nicht zu brechende Emulsion, so daß eine Extraktion nicht möglich ist. Ein leicht modifiziertes Verfahren führte zu einem öligen Rückstand, der laut HPLC-Chromatogramm durch mindestens vier Substanzen verunreinigt ist (Fig.1).

Es muß daher als ausgesprochen überraschend angesehen werden, daß sich aus biologischem Material, das wenig Galanthamin neben einer großen Anzahl an Nebenalkaloiden enthält, mit Hilfe des einfachen erfindungsgemäßen Verfahrens die freie Galanthaminbase in reiner Form und guter Ausbeute isolieren läßt, ohne daß ein zusätzlicher Adsorptionsschritt an Aluminiumoxyd erforderlich ist.

Die erfindungsgemäß hergestellte Galanthaminbase ist zur Behandlung des Engwinkelglaukoms, zur Behandlung der Alzheimerschen Krankheit oder der Alkohol- und Nikotinabhängigkeit einsetzbar. Die erfindungsgemäß hergestellte Galanthaminbase kann darüber hinaus in galenischen Zubereitungsformen, wie beispielsweise in Augensalben oder in transdermalen therapeutischen Systemen verwendet werden, die ebenfalls zur Behandlung der obengenannten Krankheiten eingesetzt werden können.

Die nachfolgenden Beispiele sollen die Erfindung erläutern, ohne sie einzuschränken:

### Beispiel 1 (Vergleich) :

Analog zum Verfahren, das in der DE-PS 11 93 061 beschrieben ist, werden 10 kg luftgetrocknete, zerkleinerte Zwiebeln von Narcissus pseudonarcissus "Carlton" sorgfältig mit 2,5 1 einer 8%igen wäßrigen Ammoniaklösung vermischt. Das Material quillt auf; der gesamte Ansatz verkleistert. Die zur Extraktion vorgesehene Zugabe von 23 1 Dichlorethan führt zu einer Emulsion, die nicht gebrochen werden kann.

### Beispiel 2 (Vergleich) :

Das im Stand der Technik bekannte Verfahren zur Isolierung des Galanthamins (DE-PS 11 93 061) wurde modifiziert. 10 kg luftgetrocknete, zerkleinerte Zwiebeln von Narcissus pseudonarcissus "Carlton" werden sorgfältig mit 400 g Natriumcarbonat vermischt. Danach werden 23 1 Dichlorethan zugegeben. Das Gemisch bleibt 10 Stunden stehen; anschließend wird das Lösungsmittel abgegossen. Die Zwiebeln werden erneut mit 23 1 Dichlorethan übergossen, das nach 2 bis 3 Stunden abgegossen wird. Im Anschluß daran werden die Zwiebeln ein drittes Mal mit 17 1 Dichlorethan versetzt, das jedoch direkt wieder abgegossen wird. Die vereinigten Dichlorethanextrakte werden mittels 10%iger Schwefelsäure extrahiert (2 x je 600 ml; 2 x je 300 ml). Die sauren Extrakte werden vereinigt und durch Ausschütteln mit Diethylether von den Spuren des Dichlorethans gereinigt. Danach werden unter Rühren und Kühlung auf 15 bis 20°C etwa 200 ml einer 25%igen wäßrigen Ammoniaklösung bis zur alkalischen Lackmusreaktion zugefügt. Der pH-Wert liegt bei 7 bis 8. Anders als im Stand der Technik angegeben fallen die Nebenalkaloide nicht aus. Die alkalische Lösung wird mit Kochsalz gesättigt und mit Diethylether extrahiert. Nach Abdampfen des Ethers bleibt, anders als im Stand der Technik angegeben, ein vernachlässigbar kleiner Rückstand übrig. Durch Sättigung mit Pottasche wird der pH-Wert der wäßrigen Phase auf etwa 14 eingestellt. Die wäßrige Phase wird mehrfach mit Diethylether extrahiert. Die vereinigten Etherextrakte werden zur Trockne eingedampft, der zurückbleibende galanthaminhaltige Rückstand in Aceton (50 ml) gelöst. Anders als im Stand der Technik angegeben, bildet sich kein Niederschlag. Man ergänzt 350 ml Aceton, fügt 200 g Aluminiumoxid hinzu und rührt 45 Minuten lang. Das Aluminiumoxid wird abfiltriert und zweimal mit je 100 ml Aceton gewaschen. Die vereinigten Acetonlösungen werden zur Trockne eingedampft. Man erhält 1,3 g eines öligen Rückstandes, der mit Hilfe der HPLC untersucht wird. Das Chromatogramm ist in Figur 1 dargestellt. Der Hauptpeak, das Galanthamin , ist gekennzeichnet. Man erkennt deutlich, daß das auf diese Weise isolierte Galanthamin durch mindestens vier Substanzen verunreinigt ist.

### Beispiel 3:

100 kg luftgetrocknete, zerkleinerte Zwiebeln von Narcissus pseudonarcissus "Carlton" werden mit 4 kg Natriumcarbonat sorgfältig vermischt. Die Mischung wird in drei gleiche Teile geteilt und mit je 15 1 Spezialbenzin 80/110 übergossen. Man läßt 24 Stunden stehen. Die Lösungsmittel werden je zweimal erneuert, gesammelt und im schwachen Vakuum zur Trockne eingeengt. Die Extrakte werden in 2%iger wäßriger Schwefelsäure aufgenommen und mit konzentrierter wäßriger Ammoniaklösung auf einen pH-Wert von 4 eingestellt. Anschließend wird fünfmal mit Diethylether extrahiert. Die wäßrige Phase wird mit konzentriertem Ammoniak auf einen pH-Wert von 9 eingestellt und fünfmal mit Diethylether extrahiert. Diese Etherfraktionen werden gesammelt, mit Natriumsulfat getrocknet und eingeengt. Es werden 20 g eines schwach gelb gefärbten öligen Rückstandes erhalten, der aus heißem Isopropanol umkristallisiert wird. Man erhält 10 g weiße Galanthaminbase mit einem Schmelzpunkt von 129 - 130°C. Im HPLC-Chromatogramm ist ausschließlich ein Peak zu erkennen (Fig.2).

## Patentansprüche

1. Pharmazeutische Zubereitung enthaltend einen reversibel wirkenden Cholinesterasehemmstoff, dadurch aekennzeichnet, daß der genannte Cholinesterasehemmstoff Galanthamin mit einer Reinheit von ≥ 99% ist.

2. Pharmazeutische Zubereitung nach Anspruch 1, **dadurch gekennzeichnet, daß** sie eine Augensalbe ist.

3. Pharmazeutische Zubereitung nach Anspruch 1, **dadurch gekennzeichnet, daß** sie ein Transdermales Therapeutisches System (TTS) ist.

4. Verwendung von Galanthamin mit einer Reinheit von ≥ 99% zur Herstellung eines Medikaments für die Behandlung eines Krankheitszustands, der durch die Verabreichung eines reversibel wirkenden Cholinesterasehemmstoffs therapierbar ist.

5. Verwendung von Galanthamin nach Anspruch 4, **dadurch gekennzeichnet, daß** der Krankheitszustand, der durch die Verabreichung eines reversibel wirkenden Cholinesterasehemmstoffs therapierbar ist, das Engwinkelglaukom ist.

6. Verwendung von Galanthamin nach Anspruch 4 oder 5, **dadurch gekennzeichnet, daß** das Medikament eine Augensalbe ist.

7. Verwendung von Galanthamin nach Anspruch 4, **dadurch gekennzeichnet, daß** der Krankheitszustand, der durch die Verabreichung eines reversibel wirkenden Cholinesterasehemmstoffs therapierbar ist, die Alzheimersche Krankheit, die Alkoholabhängigkeit, die Nikotinabhängigkeit, Poliomyelitis oder eine Curare-Applikation ist.

8. Verwendung von Galanthamin nach Anspruch 4 oder 7, **dadurch gekennzeichnet, daß** das Medikament ein Transdermales Therapeutisches System (TTS) ist.

## Claims

1. Pharmaceutical preparation containing a reversibly acting cholinesterase inhibitor, **characterized in that** the said cholinesterase inhibitor is galanthamine of a purity of ≥99%.

2. Pharmaceutical preparation according to claim 1, **characterized in that** it is an ophthalmic ointment.

3. Pharmaceutical preparation according to claim 1, **characterized in that** it is a transdermal therapeutic system (TTS).

4. The use of galanthamine of a purity of ≥99% for the production of a medicament for the treatment of a condition which is treatable by administration of a reversibly active cholinesterase inhibitor.

5. Use of galanthamine according to claim 4, **characterized in that** the condition treatable by administration of a reversibly acting cholinesterase inhibitor, is narrow-angle glaucoma.

6. Use of galanthamine according to claim 4 or 5, **characterized in that** said medicament is an ophthalmic ointment.

7. Use of galanthamine according to claim 4, **characterized in that** the condition treatable by administration of a reversibly acting cholinesterase inhibitor is Alzheimer's disease, alcohol dependence, nicotine dependence, poliomyelits or an application of curare.

8. Use of galanthamine according to claim 4 or 7, **characterized in that** the medicament is a transdermal therapeutic system (TTS).

## Revendications

1. Préparation pharmaceutique contenant un inhibiteur de la cholinestérase qui présente une action réversible, **caractérisée en ce que** l'inhibiteur de cholinestérase mentionné est la galanthamine qui possède une pureté ≥ 99 %.

2. Préparation pharmaceutique selon la revendication 1, **caractérisée en ce qu'**elle se présente sous forme de pommade ophtalmique.

3. Préparation pharmaceutique selon la revendication 1, **caractérisée en ce qu'**elle se présente sous forme de système thérapeutique transdermique (TTS).

4. Utilisation de galanthamine ayant une pureté ≥ 99 % dans la préparation d'un médicament destiné au traitement d'une maladie qui peut être traitée par administration d'un inhibiteur de la cholinestérase présentant une action réversible.

5. Utilisation de galanthamine selon la revendication 4, **caractérisée en ce que** la maladie qui peut être traitée par administration d'un inhibiteur de la cholinestérase, présentant une action réversible, est le glaucome.

6. Utilisation de galanthamine selon la revendication 4 ou 5, **caractérisée en ce que** le médicament est une pommade ophtalmique.

7. Utilisation de galanthamine selon la revendication 4, **caractérisée en ce que** la maladie, qui peut être traitée par administration d'un inhibiteur de la cholinestérase présentant une action réversible, est la maladie d'Alzheimer, l'alcoolisme, la nicotinomanie, la poliomyélite ou une administration de curare.

8. Utilisation de galanthamine selon la revendication 4 ou 7, **caractérisée en ce que** le médicament est un système thérapeutique transdermique (TTS).
